Europäisches Patentamt

European Patent Office

Office européen des brevets

(19)

(11) Publication number: **0 358 821 B1**

(12) **EUROPEAN PATENT SPECIFICATION**

(45) Date of publication of patent specification: **11.08.93**   (51) Int. Cl.⁵: **A61L 9/01**

(21) Application number: **88308467.5**

(22) Date of filing: **14.09.88**

(54) **White colored deodorizer and process for producing the same.**

(43) Date of publication of application:
**21.03.90 Bulletin 90/12**

(45) Publication of the grant of the patent:
**11.08.93 Bulletin 93/32**

(84) Designated Contracting States:
**DE FR GB IT**

(56) References cited:
**EP-A- 0 282 287**
**WO-A-81/01643**
**GB-A- 2 024 014**

(73) Proprietor: **TITAN KOGYO KABUSHIKI KAISHA**
**1978-25 Oaza Kogushi**
**Ube-shi Yamaguchi-ken(JP)**

(72) Inventor: **Kurihara, Tokumitsu**
**1-6-15-201 Higashikotoshiba**
**Ube-shi Yamaguchi-ken(JP)**
Inventor: **Saito, Tatsuo**
**6-25 Suehiro-cho**
**Ube-shi Yamaguchi-ken(JP)**
Inventor: **Harada, Hidefumi**
**3234-1 Oaza Sayama**
**Yamaguchi-shi Yamaguchi-ken(JP)**

(74) Representative: **Cropp, John Anthony David et al**
**MATHYS & SOUIRE 10 Fleet Street**
**London, EC4Y 1AY (GB)**

Rank Xerox (UK) Business Services
(3.10/3.6/3.3.1)

## Description

The present invention relates to an agent for reducing the levels of odorous gases such as ammonia, mercaptans, amines and aldehydes. More specifically, the present invention relates to a novel deodorizer in white particulate form that contains as main ingredients zinc dioxide and at least one oxide (hereinunder referred to as oxide A) selected from the group consisting of aluminium oxide, silicon oxide and mixtures thereof and which has very good adsorption characteristics.

Hydrogen sulfide, ammonia, mercaptans, amines, aldehydes and other odorous gases that are generated in daily life have caused an increasing amount of social concern about the deleterious effects they have on the biosphere and environment. In response to these concerns, a variety of deodorizers that are capable of reducing the target odorous gases have been proposed and put to practical use. Deodorizers for use in daily life have to satisfy the following minimal requirements:

(1) they must be capable of efficient reduction of the levels of hydrogen sulfide, ammonia, mercaptans, amines, aldehydes and other odorous gases that are generated in daily life;

(2) they must be safe to use;

(3) they must be easy to handle;

(4) they must be inexpensive; and

(5) they must offer a feeling of cleanliness.

However, none of the conventional deodorizers satisfy all of these requirements, nor do the most recently developed products. Activated carbon which is the most popular deodorizer in use today is highly effective in reducing mercaptans and amines but is not equally effective against hydrogen sulfide and ammonia which are typical of the odorous gases that are generated in daily life. In an attempt to solve this problem, a product has been developed that has an acid, an alkali or a certain halide supported on activated carbon. However, because of the use of an acid or alkali, this product requires very careful handling to avoid any danger to humans and hence is not suitable for daily use. Furthermore, the inherent black color of activated carbon limits the scope of use of deodorizers based on activated carbon.

Iron sulphate ($FeSO_4$) having L-ascorbic acid bound thereto is effective against basic odorous gases such as ammonia and amines but is little effective in reducing hydrogen sulfide, mercaptans and aldehydes. Furthermore, this product dissolves in water and hence is not suitable for the purpose of deodorizing wet gases.

Deodorizers classified as chemical odor modifiers are also available but many of them have strong acidity or alkalinity and kinds of odorous gases that can be effectively controlled by these odor modifiers are limited. In addition, such deodorizers are sensitive to moisture and/or a dry atmosphere.

Organic deodorizers have low heat resistance, are difficult to process, and are expensive.

It is accordingly an object of the present invention to provide a novel deodorizer that is highly effective in reducing odorous gases generated in daily life such as ammonia, mercaptans, amines and aldehydes and which is safe and easy to handle.

According to the present invention there is provided a white colored deodorizer which is a white fine powder comprising coagulated particles of (A) at least one oxide selected from the group consisting of aluminium oxide, silicon oxide and mixtures thereof and (B) zinc oxide wherein the molar ratio of zinc oxide to oxide (A) is in the range of from 2:1 to 6:1.

The invention also provides a process for producing a white colored deodorizer comprising the steps of:

combining an aqueous alkaline solution or an aqueous acidic solution with a mixed aqueous solution containing a water-soluble zinc compound and at least one compound selected from water-soluble aluminium compounds, water-soluble silicon compounds and mixtures thereof, said combining step being performed by simultaneous addition of the two solutions in such a way that the combined solution will keep its pH in the range of 6 - 12;

separating the resulting precipitate from the combined solution; and

drying the separated precipitate to form a white fine powder comprising zinc oxide and at least one oxide selected from the group consisting of aluminium oxide, silicon oxide and mixtures thereof wherein the molar ratio of zinc oxide to said at least one oxide is in the range of from 2:1 to 6:1.

Examples of the water-soluble zinc compounds that can be used as a starting material for the production of the deodorizer of the present invention include zinc sulfate, zinc chloride and zinc nitrate. Examples of suitable water-soluble aluminium compounds include aluminium sulfate, aluminium chloride and sodium aluminate. Illustrative water-soluble silicon compounds include sodium silicate and potassium silicate.

Almost any acid aqueous solution can be used for neutralizing the mixed aqueous solution, for example, sulfuric acid, hydrochloric acid, etc. The alkaline aqueous solution used for neutralizing the mixed aqueous

EP 0 358 821 B1

solution may be selected from among aqueous solutions of an alkaline metal hydroxide such as sodium hydroxide and potassium hydroxide, an alkaline earth metal hydroxide such as calcium hydroxide and barium hydroxide and an ammonia. If a sulfate is used as a zinc compound or an aluminium compound, aqueous solutions of calcium hydroxide and barium hydroxide are not desirably used since they will produce water-insoluble salts.

The water-soluble zinc compound and at least one compound selected from the group consisting of water-soluble aluminium compounds, water-soluble silicon compounds and mixtures thereof are mixed in such proportion that the molar ratio of zinc oxide to oxide A is within the range of from 2:1 to 6:1.

Zinc oxide precipitates at a pH range of 6-14, and an aluminium oxide and a silicon oxide precipitate at a pH range of 4-12 and 0-12. Accordingly, a precipitate of intended composition is formed by keeping the pH of the mixed solution between 6 and 12. If the aqueous solution contains ammonia, it is preferable that the pH of the aqueous solution is kept between 7 and 9, because zinc will make a complex salt with ammonia and will be soluble in the aqueous solution at high pH.

For the purposes of the present invention, it is essential that the mixed aqueous solution and the aqueous alkaline solution or the aqueous acidic solution be mixed together in such a way that the resulting combined solution will keep its pH between 6 and 12. If the alkaline aqueous solution or the aqueous acidic solution is added to the mixed aqueous solution gradually over a certain period of time until the combined solution has a final pH between 6 and 12, and the combined solution does not keep its pH between 6 and 12 during the addition of alkaline solution or acidic solution, the finally obtained product will not have the intended composition.

When the mixed aqueous solution and the alkaline aqueous solution are mixed together to form a precipitate composed of zinc oxide and oxide A, a temperature in the range of 20 - 80°C may be employed, with the range of 40 - 60°C being preferred.

After the precipitate has been filtered and washed, it may be dried at a temperature in the range of 100 - 350°C, preferably 120 - 250°C. The deodorizer of the present invention which contains as essential ingredients zinc oxide and oxide A will maintain satisfactory adsorption characteristics even if it is heated up to 400°C.

The white-colored deodorizer of the present invention which is based on the combination of zinc oxide and oxide A is capable of efficient reduction in the levels of hydrogen sulfide, ammonia, amines and other odorous gases that are generated in daily life. In addition, this deodorizer is safe to use since the zinc oxide and oxide A that are incorporated are nontoxic. Furthermore, the deodorizer is in a fine particulate form and can be readily supported on a carrier such as paper or other sheet materials. The deodorizer is thermally stable up to bout 400°C and can be worked into conventional plastics. In addition to the high potential of its industrial utility, the deodorizer of the present invention which is white in color is also suitable for use in cosmetics, sanitary products and disposable diapers.

The following examples are provided for the purpose of further illustrating the present invention but are not to be taken as limiting the scope thereof.

Example 1:

A 5-ℓ beaker was charged with 1 ℓ of pure water, which was heated at 60°C with stirring. A mixed aqueous solution (2 ℓ) of zinc sulfate (81.37 g as zinc oxide) and aluminium sulfate (50.98 g as aluminium oxide) and an aqueous ammonia solution were simultaneously added dropwise to the pure water in the beaker over a period of 30 minutes with care being taken to ensure that the pH of the combined solution remained at 7.5. The resulting product was filtered, washed and dried at 120°C for 6 hours to produce a white deodorizer of the zinc oxide - aluminium oxdie within the scope of the present invention.

The ability of this white deodorizer to adsorb odorous gases (i.e., hydrogen sulfide, ammonia, trimethylamine and ethyl mercaptan) was investigated by the following method. The white powder of the deodorizer (100 mg) was put into a glass vial having an inner capacity of 120 mℓ. After closing the vial with a rubber stopper, predetermined amounts of certain odorous gases were injected into the vial with a microsyringe. Two hours after the gas injection, the air in the vial was sampled with a microsyringe and the concentrations of the odorous gases in it were measured by gas chromatography. The results are shown in Table 1.

Example 2:

A white deodorizer of the zinc oxide - aluminium oxide system was prepared by repeating the procedures of Example 1 except that a mixed aqueous solution of zinc sulfate (81.37 g as zinc oxide) and

3

aluminium sulfate (25.49 g as aluminium oxide) was used. The adsorption characteristics of the prepared deodorizer for various odorous gases are shown in Table 1.

Example 3:

A 5-ℓ beaker was charged with 1 ℓ of pure water, which was heated at 60°C with stirring. A mixed aqueous solution (1 ℓ) of zinc chloride (122.05 g as zinc oxide), sodium aluminate (25.49 g as aluminium oxide) and an sodium hydroxide were simultaneously added dropwise to the pure water in the beaker over a period of 30 minutes with care being taken to ensure that the pH of the combined solution remained at 7.5. The resulting product was filtered, washed and dried at 120°C for 6 hours to produce a white deodorizer.

Example 4:

A white deodorizer was prepared by repeating the procedures of Example 3 except that the sodium aluminate was replaced by an aqueous solution of sodium silicate (30.04 g as silicon oxide).

Table 1

| Composition (molar ratio) | Gas concentration (ppm) after 30 min of adsorption | | | |
| --- | --- | --- | --- | --- |
| | Ammonia 1000* | Hydrogen sulfide 1000* | Ethyl mercaptan 1000* | Trimethyl amine 1000* |
| Example 1 ZnO:Al$_2$O$_3$=2:1 | 30 | 0 | 5 | 20 |
| Example 2 "=4:1 | 10 | 0 | 2 | 30 |
| Example 3 "=6:1 | 5 | 0 | 0 | 10 |
| Example 4 ZnO:SiO$_2$=3:1 | 0 | 0 | 0 | 10 |

*Initial concentrations in ppm.

## Claims

1. A white colored deodorizer which is a white fine powder comprising coagulated particles of (A) at least one oxide selected from the group consisting of aluminium oxide, silicon oxide and mixtures thereof and (B) zinc oxide, wherein the molar ratio of zinc oxide to oxide (A) is in the range of from 2:1 to 6:1.

2. A process for producing a white colored deodorizer comprising the steps of:
combining an aqueous alkaline solution or an aqueous acidic solution with a mixed aqueous solution containing a water-soluble zinc compound and at least one compound selected from water-soluble aluminium compounds, water-soluble silicon compounds and mixtures thereof, said combining step being performed by simultaneous addition of the two solutions in such a way that the combined solution will keep its pH in the range of 6 - 12;
separating the resulting precipitate from the combined solution; and
drying the separated precipitate to form a white fine powder comprising zinc oxide and at least one oxide selected from the group consisting of aluminium oxide, silicon oxide and mixtures thereof wherein the molar ratio of zinc oxide to said at least one oxide is in the range of from 2:1 to 6:1.

3. A process according to Claim 2 wherein the reaction for forming said precipitate is carried out at a temperature of 20 - 80 °C, and the resulting precipitate is dried at a temperature of 120 - 250 °C.

**Patentansprüche**

1. Weißes Desodorierungsmittel, das ein weißes feines Pulver ist, umfassend koagulierte Partikel aus (A) mindestens einem Oxid, ausgewählt aus der Gruppe, bestehend aus Aluminiumoxid, Siliciumoxid und deren Mischungen, (B) Zinkoxid, wobei das Molverhältnis von Zinkoxid zu Oxid (A) im Bereich von 2:1 bis 6:1 liegt.

2. Verfahren zur Herstellung eines weißen Desodorierungsmittels, umfassend folgende Schritte:
Vereinigen einer wässrigen alkalischen Lösung oder einer wässrigen sauren Lösung mit einer wässrigen Lösungsmischung, die eine wasserlösliche Zinkverbindung und mindestens eine Verbindung enthält, die ausgewählt ist aus wasserlöslichen Aluminiumverbindungen, wasserlöslichen Siliciumverbindungen und deren Mischungen, wobei dieser Vereinigungsschritt ausgeführt wird durch gleichzeitige Zugabe der beiden Lösungen in einer solchen Weise, daß der pH der vereinigten Lösung im Bereich von 6 bis 12 gehalten wird;
Abtrennen des resultierenden Niederschlags von der vereinigten Lösung; und
Trocknen des abgetrennten Niederschlags zur Bildung eines weißen feinen Pulvers, das Zinkoxid und mindestens ein Oxid umfaßt, das ausgewählt ist aus der Gruppe bestehend aus Aluminiumoxid, Siliciumoxid und deren Mischungen, worin das Molverhältnis von Zinkoxid zu jenem mindestens einen Oxid im Bereich von 2:1 bis 6:1 liegt.

3. Verfahren gemäß Anspruch 2, worin die Reaktion zur Bildung des Niederschlags bei einer Temperatur von 20-80 °C ausgeführt wird und der resultierende Niederschlag bei einer Temperatur von 120-250 °C getrocknet wird.

**Revendications**

1. Désodorisant de couleur blanche qui est une fine poudre blanche comprenant des particules coagulées de (A) au moins un oxyde choisi dans le groupe formé par l'oxyde d'aluminium, l'oxyde de silicium et leurs mélanges, et (B) oxyde de zinc, dans lequel le rapport molaire de l'oxyde de zinc à l'oxyde (A) se situe dans l'intervalle de 2:1 à 6:1.

2. Procédé de production d'un désodorisant de couleur blanche comprenant les étapes suivantes :
combiner une solution alcaline aqueuse ou une solution acide aqueuse avec une solution aqueuse mixte contenant un composé de zinc hydrosoluble et au moins un composé choisi parmi les composés d'aluminium hydrosolubles, les composés de silicium hydrosolubles et leurs mélanges, ladite étape de combinaison étant exécutée par addition simultanée des deux solutions de telle manière que le pH de la solution combinée soit maintenu dans l'intervalle de 6 à 12 ;
séparer le précipité résultant de la solution combinée ; et
sécher le précipité séparé pour former une fine poudre blanche comprenant de l'oxyde de zinc et au moins un autre oxyde choisi dans le groupe formé par l'oxyde d'aluminium, l'oxyde de silicium et leurs mélanges, dans laquelle le rapport molaire de l'oxyde de zinc audit ou auxdits autre(s) oxyde(s) se situe dans l'intervalle de 2:1 à 6:1.

3. Procédé selon la revendication 2, dans lequel la réaction de formation dudit précipité est conduite à une température de 20 à 80°C, et le précipité résultant est séché à une température de 120 à 250°C.